# EUROPEAN PATENT SPECIFICATION

(11) **EP 2 939 810 B1**
(45) Date of publication and mention of the grant of the patent: **06.06.2018**
(21) Application number: 13868960.9
(22) Date of filing: 22.04.2013
(51) Int. Cl.: F04B 13/00, F04B 53/14, F04B 53/16, B24B 1/00, B24B 37/02, B24B 5/50, B28D 5/00, A61M 5/168, C30B 33/00, C30B 29/20, A61M 5/142, A61M 5/145

(54) **METHOD FOR PROCESSING CYLINDRICAL SURFACES OF SAPPHIRE PARTS, A SAPPHIRE PISTON PAIR AND A METERING PUMP ON THE BASIS THEREOF**
VERFAHREN ZUR VERARBEITUNG VON ZYLINDRISCHEN OBERFLÄCHEN VON SAPHIRTEILEN, SAPHIRKOLBEN UND DARAUF BASIERENDE DOSIERPUMPE
PROCÉDÉ DE TRAITEMENT DE SURFACES CYLINDRIQUES DE PIÈCES À BASE DE SAPHIR, PAIRE DE PISTON AU SAPHIR ET POMPE-DOSEUR LA COMPRENANT

(30) Priority: 27.12.2012 RU 2012157503
(43) Date of publication of application: 04.11.2015
(73) Proprietor: Obschestvo S Ogranichennoy Otvetstvennostyu "Farmasapfir", Moscow 121354 (RU)
(72) Inventor: SAVENKOV, Vitaly Alekseevich, Moscow, 121351 (RU)
(74) Representative: Glawe, Delfs, Moll
(86) International application number: PCT/RU2013/000342
(87) International publication number: WO 2014/104925

(56) References cited:
- EP-A1- 2 461 379
- JP-A- 2004 060 513
- RU-C1- 2 240 733
- RU-C2- 2 314 762
- SU-A1- 1 112 043
- UA-A- 48 581
- US-A- 4 405 294
- US-B1- 6 375 551

## Description

The set of inventions is related to devices, particularly, to pump elements and pump-element-based dosing pumps, and also to fabricating devices and parts of these, particularly, to the sapphire parts' cylindrical surface machining. The group of inventions can be used in any technical field wherein pump elements are utilized, including as pumping and/or dosing devices (dosing pumps) components, particularly, in pharmaceutical, food processing, chemical, perfumery, cosmetic, engineering and other industries.

Now, most of the dosing pumps for pharmaceutical and food processing industries comprise pump elements of various metallics and ceramics (see, for example, US4273263 A, 16.06.1981; DE2723320 C2, 04.11.1982; FR2797046 A1, 02.02.2001) .

But, for dosing pumps with metal and ceramic pump elements, there is the problem of rubbing parts. Smallest particles of these parts' material are formed due to contacting parts' friction contaminate the liquids being dosed, which is especially undesirable in the pharmaceutical industry. Also, due to rubbing parts wearing down, the volume being dosed changes, which is also not permissible for high-precision dosing. Moreover, dosing pumps in pharmaceutical and food processing industries should stand long-term exposure to aggressive operating factors, in particular, the sterilization process.

A significantly higher wear resistance can be achieved with the fabrication of pump element parts of crystals, in particular, of crystals based upon α-modification of aluminium oxide (α-Al₂O₃ aka corundum) .

The performed study has shown that, e.g. leucosapphire (being the version of α-Al₂O₃) directed towards the reference axis [0001] has a wear resistance 10 times higher than that of chromium coating, and 5 times higher than that of corundum ceramics.

In addition, leucosapphire is transparent within the wide wavelength domain, it has poor light-scattering and high optical homogeneity, high radiation resistance and low internal stress, and high aggressive media resistance. Leucosapphire transparency is the additional advantage lying in possibility to visually monitor the bubbles' presence/absence during dosing pumps' operation, which is important for high-precision dosing.

As technology dictates, the pump element's parts are made of leucosapphire crystal (RU2240733 C1, 27.11.2004). This one is the present invention's prototype. This prototype's disadvantage is high surface roughness. According to an assessment by the present invention's author made on the basis of knowledge used in the engineering solution of sapphire pump element parts' cylindrical surface machining method, the prototype sapphire pump element parts' surface roughness Ra exceeds 5 µm. High surface roughness of rubbing parts leads to increased friction force between them that in turn results in increased wear of rubbing parts and correspondingly, impacts the operation consistency and life of pump elements and pump-element-based dosing pumps.

The task that this group of inventions is to solve lies in development of sapphire cylindrical surfaces' machining method allowing to obtain surface roughness level Ra of 0.2-0.5 nm (2-5 Å) and fabrication by this method of pump element of sapphire with minimum friction coefficient.

The pump element consists mainly of leucosapphire single crystal, but can be fabricated also of crystals based upon α-modification of aluminium oxide (α-Al₂O₃) with admixtures, e.g. of alexandrite-colour crystal, of red ruby, of blue sapphire, of orange sapphire, of orange padparadscha, of yellow sapphire, of green sapphire, of rose sapphire, of dark-red sapphire, of violet sapphire and others.

The invention relates to a method of machining cylindrical surfaces of parts of aluminium-oxide-α-modification -based crystal according to claim 1 and a pump element comprising at least one external and internal part of aluminium-oxide-α-modification - based crystal according to claim 7. Made of cultured leucosapphire crystal, preliminary workpieces with the allowances required for further machining are drilled using a diamond tool (forming). The main assembly units of sapphire pumping and dosing devices are as follows: the external portion (part) is the sapphire barrel (aka case, sleeve or liner) and the internal portion (part) is the sapphire plunger (aka piston or ram). Sapphire barrel and sapphire plunger together form the pump element. Thus, the pump element comprises at least one external part of aluminium-oxide-α-modification-based crystal and at least one internal part of aluminium-oxide-α-modification-based crystal. For some models of dosing pumps, the design provides for sapphire slide gate (the second plunger acting as stopper valve).

Preliminary cylindrical sapphire workpieces can be obtained by two different methods. In the first method, cylindrical workpieces of sapphire piston and barrel are drilled from one-piece cultured sapphire single crystal. Drilling is performed with diamond drill of the required diameter with due account for allowance for further machining. Drilling is performed using various lubricating fluids. These can be various grades of synthetic cutting oils, e.g. 5W-ADDINOL Super light 5W-40, OW CASTROL Formula SLXOW-30 and others. The remaining material is returned to the crystal melting and growth plant. The second method lies in sawing of one-piece sapphire "boule" (growth by Kyropoulos technique) or "keel" (growth by Bagdasarov technique) monolith with diamond discs to obtain square blocks. Then, these blocks are machined at grinding machining equipment with coarse-grained diamond discs, i.e. rough grinding is performed. The result is sapphire cylindrical rough-machined workpieces. The first method is preferable.

Then, the workpieces are machined at high-precision machining equipment using diamond tools. In the process, various diamond discs, diamond drills and diamond surrounded hones are used with various diamond grain sizes. In all the machining processes various lubricating fluids are used compulsorily. These can be various grades of synthetic cutting oils, e.g. 5W-ADDINOL Super light 5W-40, OW CASTROL Formula SLXOW-30 and others.

Then, the sapphire workpieces undergo machining with diamond tools of smaller diamond grains. At this machining stage, minimum flaw allowance is achieved for subsequent more precise and fine machining stages. The process can be performed on grinding equipment of various kinds and modifications, in particular, on multi-purpose circular grinding machine of CG 2535-AL or CG 2550-AL model, multi-purpose circular grinding semi-automated machine of 3U12AAF11 model with Digital Readout Unit, and others.

Then, pre-finish machining of leucosapphire workpieces is performed with the minimum grain size diamond tools. Diamond discs at Ml binder are used with grain size of 125/100 um, 100/80 µm. Content is 100%, diamond grade is AC 15, AC 20, AC 32, tool speed is 5 m/sec. In the process, the surface roughness Ra of 0.6 um is achieved, and the outer layer depth is h = 11 um. The product removal speed reaches 1,000 µm/min. This machining stage is performed step-by-step using cutting diamond tools with constant reduction of these tools' diamond grain size. Depending on the certain part being machined, machining can be performed both on centreless equipment and between centres, as anyone skilled in the art knows.

When single crystals make contact with machining tools, anisotropy of leucosapphire properties must be taken into account. Machining quality improvement is achieved due to reduction of cutting with single grains of a tool.

Upon completion of pre-finish machining, the generated internal stress must be relieved in sapphire workpieces in order sapphire cracking wouldn't occur. For this, the sapphire workpiece annealing technique is used. The workpiece is loaded into a muffle furnace where at step based heating to 700-800 °C for 20-30 minutes, internal stress relief occurs. Then the furnace is turned off for slow cooling. The cooled workpieces are ready for further finish machining.

Next comes the finish machining, or chemical and mechanical polishing. Surface roughness of Ra of 0.2-0.5 nm (2-5 Å) of working faces of leucosapphire pump elements in sapphire dosing pumps is achieved as follows. The parts having undergone prefinish machining undergo chemical and mechanical polishing. Quasi-moldability mode is used allowing machining of surfaces with roughness of 2-10 nm, removal is 0.4 µm/ min, and machining is performed with cloth polishing wheel with diamond grain of 5/3 µm on clock oil at pressure of 1.5*10⁻² KSS/mm²; in fine polishing, machining is performed with 1/0 µm diamond grain on clock oil, removal is 0.1 µm/min, and the result of the process is so-called nanoscale surface texture. As a finishing tool, semi-soft and soft laps and polishing wheels are used. The working part of these tools is made of semi-soft and soft materials such as polyurethane, chamois, felt, cloth; the most preferable materials are genuine chamois and cloth. As polishing material, highly purified filtered clock grades of oils are used.

During chemical and mechanical polishing of cylindrical sapphire surfaces, the intermediate layer formed due to fluids' chemical action is removed rather than the base material. The etching process can be divided into the two following stages:
- diffusion of anion and cation complexes on the crystal surface;
- adsorbed compound formation and removal from the crystal surface.

Roughness of machined surfaces reduces and their texture and purity improves:
- as diamond suspension grain size reduces;
- at transfer from solid grinding tools in rough and fine polishing operations to flexible and soft polishing instruments of polyurethane, chamois and felt in final machining operations;
- at transfer from diamond abrasive cutting to tribochemical interaction of polishing compound, e.g. NALCO-2354, NALCO 2360 or Siopol-1 (V.V. Rogov. Physicochemistry in Processes of Formation of Functional Surfaces of Electronic Equipment and Optical Systems' Parts of Glass and Sapphire (α-Al2O3) at Tribochemical Polishing. Sverkhtvyordiye materialy, 2009, No.4, pp.74-83; UA48581 A, 15.08.2002), with sapphire at allowance removal.

In the process, minimum roughness is achieved Ra of 2÷5 Å of machined cylindrical surface and the highest class optical purity P 0-10 (State Standard (GOST) 11141-84). With that, the rate of etching solution supplied drop by drop to the polishing wheel is 25-45 drops per minute. To obtain such results, one of two types of polishing compounds is used:
1. Siopol-1 with water-ammonia colloidal liquor OSCh.6-3, pH 9.3 (V.V. Rogov. Physicochemistry in Processes of Formation of Functional Surfaces of Electronic Equipment and Optical Systems' Parts of Glass and Sapphire (α-Al2O3) at Tribochemical Polishing. Sverkhtvyordiye materialy. 2009, No.4, pp.74-83; UA48581 A, 15.08.2002)
2. Colloidal silica water solution (S1O2) NALCO-2354, pH 10.85 or NALCO-2360, pH 8.54.

Surface roughness Ra is 0.2-0.5 nm (2-5 Å). All the machining processes, their high geometric accuracy for roundness, parallelism, centricity, linearity and surface roughness at all fabrication stages are tracked on electronic screens of high-precision machining equipment monitors. Final acceptance for fabrication quality is performed in the QCD laboratory using mechanical and electronic measuring instruments. Ra parameter is measured using optical noncontact instruments, in particular, MII-5, MII-10 and MII-15 (MII stands for measuring interference microscope), MPI (roughness-indicating interference microscope) and in double-beam analysing system FEI Helios 650 Nanolab for scanning electronic and ionic microscopy(SEM: Surface microscopy with resolution of less than 0.7 nm).

Then, if necessary, sapphire ram and barrel are pressed on the appropriate metal parts using a manual mechanical press. Auxiliary portions of dispensers, in particular, of dispensers for attachment to a machine, for pressing of sapphire on metal, consist of medical-grade stainless steel preferably of AISI 316 L grade or 12Kh18N10T grade.

By the above described "multi-step method of chemical and mechanical polishing" of sapphire pump elements' working faces the invention technical result is achieved, that is rubbing surfaces' friction coefficient reduction that in turn allows reducing of pump element parts' wear thus increasing the pump-element-based dosing pump service life including prolonged maintaining of dispensed fluids' dosing accuracy that is especially important in pharmaceutical industry when liquid medicinal products' dosing. The expected service life of dosing pumps based upon machined by "multi-step method of chemical and mechanical polishing" sapphire pump elements is 25-30 years that significantly exceeds service life of the currently commercial-off-the-shelf dosing pumps with pump elements of metallics and ceramics.

The above exemplary embodiment is non-limiting, and other embodiments within the claims can be implemented by a person skilled in the art. For the claimed pump elements and the pump-element-based dosing pump, the only essential features in the cause and effect relationship with the technical result being achieved are the material the pump element parts are made of - the aluminium-oxide-α-modification-based crystal, preferably the leucosapphire single crystal, and surface roughness Ra of 0.2-0.5 nm (2-5 Å); all other features such as presence of additional parts, geometry and materials of parts except for material of barrel and piston, design features of dosing pumps, etc. are unessential to achieve the claimed technical result, and can be selected by a person skilled in the art from known options on the basis of known design principles. For the claimed method of machining of cylindrical surfaces of parts of aluminium-oxide-α-modification-based crystal with the achievement of surface roughness Ra of 0.2-0.5 nm (2-5 Å) the essential features in the cause and effect relationship with the technical result being achieved are presence of the following stages and substances: drilling of preliminary workpieces of parts of aluminium-oxide-α-modification-based crystal using diamond tool, three-step machining of surface with diamond tool in the presence of lubricating fluids with successive reduction of abrasive grain size to 125/100 and/or 100/80 um, relieving of internal stress in workpieces through annealing in the muffle furnace, surface machining with semi-soft or soft polishing wheel or lap with diamond grain size of 5/3 um and/or 1/0 um on clock oil, surface tribochemical polishing with colloidal-SiO₂-based polishing compound. The present method of machining can be used for other non-flat surfaces of sapphire, too, e.g. for spherical surfaces.

The set of inventions meets the unity of invention requirement since the machining method is intended for fabrication of the pump element and the pump-element-based dosing pump, and the pump element is intended for use in the dosing pump.

## Claims

1. The method of machining cylindrical surfaces of parts of aluminium-oxide-α-modification-based crystal with achievement of surface roughness Ra of 0.2-0.5 nm (2-5 Å) comprising: drilling of preliminary workpieces of parts of aluminium-oxide-α-modification-based crystal using diamond tool, three-step machining of the surface with the diamond tool in the presence of lubricating fluids with successive reduction of abrasive grain size to 125/100 and/or 100/80 µm, relieving of internal stress in workpieces through annealing in the muffle furnace, surface machining with semi-soft or soft polishing wheel or lap with diamond grain size of 5/3 µm and/or 1/0 µm on clock oil, surface tribochemical polishing with colloidal-SiO₂-based polishing compound.

2. The method of claim 1, wherein aluminium-oxide-α-modification-based crystal is the leucosapphire single crystal.

3. The method of claim 1, wherein aluminium-oxide-α-modification-based crystal is the crystal with admixtures selected from the following group: alexandrite-colour crystal, red ruby, blue sapphire, orange sapphire, orange padparadscha, yellow sapphire, green sapphire, dark-red sapphire, violet sapphire.

4. Method of any of claims 1-3, wherein the parts are the pump element barrel and piston.

5. The method of claim 1, wherein working part of semi-soft or soft polishing disc or lab is made of polyurethane, chamois, felt or cloth.

6. The method of claim 1, wherein NALCO-2354, NALCO-2360 or Siopol-1 is used as the colloidal-SiO₂-based polishing compound.

7. A pump element comprising at least one external part of aluminium-oxide-α-modification-based crystal and at least one internal part of aluminium-oxide-amodification-based crystal; **characterised in that** a roughness Ra of contacting working faces of the parts is 0.2-0.5 nm (2-5 Å).

8. The pump element of claim 7, wherein aluminium-oxide-α-modification-based crystal is the leucosapphire single crystal.

9. The pump element of claim 7, wherein aluminium-oxide-α-modification-based crystal is the crystal with admixtures selected from the following group: alexandrite-colour crystal, red ruby, blue sapphire, orange sapphire, orange padparadscha, yellow sapphire, green sapphire, dark-red sapphire, violet sapphire.

10. The pump element of any of claims 7-9, wherein the external and the internal parts are the barrel and the piston, correspondingly.

11. A dosing pump comprising the pump element of claim 7.

12. The dosing pump of claim 11 for utilization in pharmaceutical, food processing, chemical, perfumery, cosmetics or engineering industry.

## Patentansprüche

1. Verfahren zur Bearbeitung von zylindrischen Oberflächen von Teilen aus Kristall auf der Basis von α-modifizierten Aluminiumoxid mit einer Oberflächenrauigkeit Ra von 0,2-0,5 nm (2-5 Å), umfassend:
Bohren von Vorformen von Werkstücken aus Kristall auf der Basis von α-modifizierten Aluminiumoxid mit einem Diamantwerkzeug, dreistufige mechanische Behandlung der Oberfläche mit dem Diamantwerkzeug in Anwesenheit eines Schmiermittels mit sukzessiver Verringerung der Schleifkorngröße auf 125/100 und/oder 100/80 µm, Aufhebung der inneren Spannungen in den Werkstücken mittels Tempern in einer Muffelofen, Oberflächenbehandlung mit einer halbweichen oder weichen Polierscheibe oder Lappen mit einer Diamantkörnung von 5/3 µm und/oder 1/0 µm mit Uhrenöl, tribochemische Polierung der Oberfläche mit einer Polierzusammensetzung auf der Basis von kolloidalem SiO₂.

2. Verfahren nach Anspruch 1, wobei der Kristall auf der Basis von α-modifizierten Aluminiumoxid ein Leukosaphir-Einkristall ist.

3. Verfahren nach Anspruch 1, wobei der Kristall auf der Basis von α-modifizierten Aluminiumoxid ein Kristall mit Zusätzen ist, ausgewählt sind aus der Gruppe bestehend aus: Alexandrit-Kristall, rotem Rubin, blauem Saphir, orangem Saphir, orangem Padaraz, gelbem Saphir, grünem Saphir, dunkelrotem Saphir, violettem Saphir.

4. Verfahren nach einem der Ansprüche 1-3, wobei die Teile ein Zylinder und ein Kolben eines Kolbenpaars sind.

5. Verfahren nach Anspruch 1, wobei das Arbeitsteil der halbweichen oder weichen Polierscheibe oder Lappen aus Polyurethan, Wildleder, Filz oder Stoff hergestellt ist.

6. Verfahren nach Anspruch 1, wobei NALCO-2354, NALCO-2360 oder Siopol-1 als Polierzusammensetzung auf der Basis von kolloidalem SiO₂ verwendet wird.

7. Pumpenelement umfassend mindestens ein Außenteil eines Kristalls auf der Basis von α-modifizierten Aluminiumoxid und mindestens ein Innenteil eines Kristalls auf der Basis von α-modifizierten Aluminiumoxid, **dadurch gekennzeichnet, dass** die Rauheit Ra der Arbeitskontaktflächen der Teile 0,2-0,5 nm (2-5 Å) beträgt.

8. Pumpenelement nach Anspruch 7, wobei der Kristall auf der Basis von α-modifizierten Aluminiumoxid ein Leukosaphir-Einkristall ist.

9. Pumpenelement nach Anspruch 7, wobei der Kristall auf der Basis von α-modifizierten Aluminiumoxid ein Kristall mit Zusätzen ist, ausgewählt aus der Gruppe bestehend aus Alexandrit-Kristall, rotem Rubin, blauem Saphir, orangem Saphir, orangem Padaraz, gelbem Saphir, grünem Saphir, dunkelrotem Saphir, violettem Saphir.

10. Pumpenelement nach einem der Ansprüche 7-9, wobei das Außenteil und Das Innenteil jeweils Zylinder und Kolben sind.

11. Pumpspender umfassend ein Pumpenelement nach Anspruch 7.

12. Pumpenspender nach Anspruch 11 zur Verwendung in der pharmazeutischen, Lebensmittel-, chemischen, Parfüm-, Kosmetik- oder Maschinenbauindustrie.

## Revendications

1. Procédé d'usinage de surfaces cylindriques de pièces en cristal basé sur la modification α de l'oxyde d'aluminium avec obtention d'une rugosité de surface Ra de 0,2-0,5 nm (2-5 Å) comprenant :
le perçage de pièces préliminaires de fabrication de pièces en cristal basé sur la modification α de l'oxyde d'aluminium au moyen d'un outil en diamant, l'usinage en trois étapes de la surface avec l'outil en diamant en présence de fluides lubrifiants avec réduction successive de la taille des grains d'abrasif jusqu'à 125/100 et/ou 100/80 µm,
le relâchement des contraintes internes dans les pièces de fabrication par recuit dans le four à moufle,
l'usinage de la surface avec une meule ou un disque de polissage semi-doux ou doux avec une taille de grains de diamant de 5/3 µm et/ou 1/0 µm sur de l'huile d'horlogerie, le polissage tribochimique de la surface avec un composé de polissage à base de SiO₂ colloïdal.

2. Procédé de la revendication 1, dans lequel le cristal basé sur la modification α de l'oxyde d'aluminium est le monocristal de leucosaphir.

3. Procédé de la revendication 1, dans lequel le cristal basé sur la modification α de l'oxyde d'aluminium est le cristal avec des mélanges choisis dans le groupe suivant : cristal de la couleur de l'alexandrite, rubis rouge, saphir bleu, saphir orange, padparadscha orange, saphir jaune, saphir vert, saphir rouge foncé, saphir violet.

4. Procédé de l'une quelconque des revendications 1 à 3, dans lequel les pièces sont le cylindre et le piston d'un élément de pompe.

5. Procédé de la revendication 1, dans lequel la partie active de la meule ou du disque de polissage semi-doux ou doux est constituée de polyuréthane, de peau de chamois, de feutre ou de tissu.

6. Procédé de la revendication 1, dans lequel le NALCO-2354, le NALCO-2360 ou le Siopol-1 est utilisé comme le composé de polissage à base de SiO₂ colloïdal.

7. Élément de pompe comprenant au moins une pièce externe en cristal basé sur la modification α de l'oxyde d'aluminium et au moins une pièce interne en cristal basé sur la modification α de l'oxyde d'aluminium ; **caractérisé en ce qu'**une rugosité Ra des faces de travail en contact des pièces est de 0,2-0,5 nm (2-5 Å).

8. Élément de pompe de la revendication 7, dans lequel le cristal basé sur la modification α de l'oxyde d'aluminium est le monocristal de leucosaphir.

9. Élément de pompe de la revendication 7, dans lequel le cristal basé sur la modification α de l'oxyde d'aluminium est le cristal avec des mélanges choisis dans le groupe suivant : cristal de la couleur de l'alexandrite, rubis rouge, saphir bleu, saphir orange, padparadscha orange, saphir jaune, saphir vert, saphir rouge foncé, saphir violet.

10. Élément de pompe de l'une quelconque des revendications 7 à 9, dans lequel les pièces externe et interne sont respectivement le cylindre et le piston.

11. Pompe de dosage comprenant l'élément de pompe de la revendication 7.

12. Pompe de dosage de la revendication 11 pour utilisation dans l'industrie pharmaceutique, agroalimentaire, chimique, de la parfumerie, cosmétique ou mécanique.
